# EUROPEAN PATENT APPLICATION

(11) **EP 3 081 242 A1**
(43) Date of publication of application: **19.10.2016**
(21) Application number: 15305551.2
(22) Date of filing: 14.04.2015
(51) Int. Cl.: A61M 1/02, A61J 3/00, G01N 33/49

(54) **System for the production and storage of autologous serum**

(71) Applicant: Eyechor Medical, 75019 Paris (FR)
(72) Inventor: Roy, Pierre, 75019 Paris (FR); Foschini, Fulvio, 00124 Rome (IT)
(74) Representative: Regimbeau

(57) **Abstract**

The proposed invention relates to a system for preparing serum or plasma from a blood sample, comprising a main unit (1), comprising
o a sampling chamber (10) for receiving a blood sample, and
o a filtration chamber (20) for filtering the blood sample, the filtration chamber comprising a first compartment (21) in fluid communication with the sampling chamber, a second compartment (23), and a filter (22) interposed between the first and second compartments,
characterized in that it further comprises a plurality of vials, each comprising a connector, and the main unit comprises a plurality of connectors (30) adapted to cooperate with the vial connectors to achieve a leak-tight communication between the main unit and the vials,
and in that the filtration chamber is designed to allow blood filtration by gravity when the main unit is in a position of use.

## Description

### TECHNICAL FIELS OF THE INVENTION

The invention relates to a device for preparation of autologous serum or plasma from a blood sample, and its storage in a number of separate vials.

The invention can in particular be implemented for the preparation and storage of eye drops.

### BACKGROUND ART

Serum is the fluid component of blood that remains after clotting.

It is currently used for the treatment of a number of pathologies, especially in the form of eye drops for treating eye injuries or syndromes, such as persistent epithelial defects, superficial punctate keratitis, dry eye (sicca syndrome) or Sjögren's Syndrome, neutrophic keratopathy, ocular Graft versus Hot Disease (GvHD), recurrent erosion syndrome, superior limbal keratoconjunctivitis (SLK), and chemical injuries or burns.

Serum eye drops can also be used during ocular surface surgery or during a postoperative period after ocular surface surgery (limbal stem cell transplantation, penetrating keratoplasty, refractive surgery, etc.)

Serum can also be used intraocularly during vitrectomy in diabetic patients or trabeculectomy.

Serum can be obtained from a blood sample. In particular, autologous serum can be obtained by processing one's own blood sample.

Various protocols exist for preparing serum from a blood sample. An example of preparation comprises the following steps:
- Sampling a quantity of blood comprised between 30 mL and 470 mL,
- Allowing the blood sample to clot for a duration comprised between 15 minutes and 24 hours,
- Centrifuging the clotted blood sample to separate serum from the clotted blood components - the clotting step increasing the efficiency of the centrifuging step,
- Optionally diluting the obtained serum to a concentration comprised between 20 and 100%,
- Optionally filtering the solution, and
- Filling a plurality of vials or aliquots with the solution.

The devices currently available for manufacturing, storing, transporting and delivering serum to a patient are so-called non-closed devices, in the sense that these devices always need to be opened apart at least once from the blood collection to the step of application to the patient, thereby putting in contact the serum with the atmosphere.

Therefore, in order to respect the regulations of the European Pharmacopoeia, eye drops have always to be produced under sterile conditions. The manufacture of serum eye drops therefore requires that a blood sample or donation be submitted to a clean room laboratory where it is processed into serum, and subsequently returned to an attending physician for delivery to a patient.

This procedure is time-consuming and expensive, and makes it impossible for a blood establishment, for instance, to product autologous serum itself, as long as this establishment has no clean room laboratory.

### SUMMARY OF THE INVENTION

One aim of the invention is to overcome the aforementioned disadvantages of the prior art and provide a device which allows production and conditioning of serum from a blood sample within said device under continuous closure of said device, thereby avoiding the need for a clean room laboratory.

Another aim of the invention is to allow production and conditioning of serum without the need of auxiliary means or any source of energy.

Another aim of the invention is to also allow production and conditioning of plasma, that is blood serum enriched with clotting factors.

Another aim of the invention is to allow production of serum or plasma from a whole blood donation, i.e. from blood extracting from a human or animal, containing all native blood components.

Another aim of the invention is to render the production of serum quick and inexpensive.

According to the invention, a system for preparing serum or plasma from a blood sample is provided, comprising a main unit, comprising
o a sampling chamber for receiving a blood sample, and
o a filtration chamber for filtering the blood sample, the filtration chamber comprising a first compartment in fluid communication with the sampling chamber, a second compartment, and a filter interposed between the first and second compartments,
characterized in that it further comprises a plurality of vials, each comprising a connector, and the main unit comprises a plurality of connectors adapted to cooperate with the vial connectors to achieve a leak-tight communication between the main unit and the vials,
and in that the filtration chamber is designed to allow blood filtration by gravity when the main unit is in a position of use.

In some embodiments, the system may comprise the following features :a pair of connectors, comprising a connector of a vial and a connector of the main unit, may be designed such that:
∘ the opening of one connector causes the other connector to open, and
∘ the opening of a connector occurs only if the vial is in contact with the main unit.
   - each connector of a pair of connectors comprising a vial connector and a main unit connector, may comprise a wall comprising a through hole, and a plug adapted to close said through hole, movably mounted relative to the respective wall so as to selectively close or open the respective through hole.
   - at least one plug of the pair of connectors may be mounted in translation relative to the respective wall.
   - at least one plug of the pair of connectors may be a flexible plate covering the respective hole and fastened to the wall by at least one fixation point. The fixation point may be located on a side edge of the flexible plate, or on a center point of the flexible plate.
   - At least one connector of the pair of connectors may comprise a protruding element extending outwardly from the respective wall and adapted to cause the plug of the other connector to move when the vial is brought in contact with the main unit. At least one protruding element of the pair of connectors may comprise a pin protruding from a connector's plug. At least one protruding element of the pair of connectors may also comprise a tube protruding from the respective wall. In an embodiment, the protruding elements of both connectors of the pair are tubes protruding from the respective walls, and the tubes are offset relative to their respective through hole by an equal distance thereby allowing each tube of a connector to penetrate in the other connector's through hole at the same time.
   - each connector of the pair of connectors may comprise a plug having at least one aperture, the plug being rotatably mounted with respect to the respective wall, allowing selective alignment of the plug aperture with the respective through hole, and the plugs having coupling means allowing the rotation of a plug to cause the other plug of the pair to rotate when the vial is brought in contact with the main unit.
   - Each main unit connector may comprise a needle and a sheath covering the needle, and each vial connector may comprise a septum, the needle being adapted to pierce both the sheath and the septum.
   - The filtration chamber may comprise a dispensing plate designed to collect filtrated blood, and the dispensing plate may comprise a plurality of cavities of equal capacity, the cavities having coplanar bottom walls, and being fluidly connected to each other by communication channels extending at a constant distance from the plan of the bottom walls to ensure equal distribution of a quantity of fluid to the cavities.
   - The main unit may further comprise a supplementation plate comprising a plurality of compartments, the supplementation plate bearing the main unit connectors and being connected to the dispensing plate so that each compartment of the supplementation plate is fluidly connected with a cavity of the dispensing plate.
   - When the main unit is in position of use, the sampling chamber may extend above the filtration chamber, the first compartment extends above the second compartment and the dispensing plate extends below the filter, thereby allowing blood filtration by gravity.

A process for manufacturing serum or plasma from a blood sample is also provided, the process being carried out in a system according to the above description, and comprising:
- a step of providing a blood sample in a sampling chamber,
- filtrating the blood sample by gravity in the filtration chamber,
- connecting a plurality of vials to the main unit, and
- filling the vials with the serum or plasma obtained by blood filtration.

The system according to the invention allows manufacturing serum or plasma, that is, platelets-enriched serum, from a blood sample without the need to expose the blood or serum to the atmosphere, and thus without the need for a clean room laboratory to prevent contamination.

Indeed, once the sampling chamber of the system is filled with a blood sample, the filtration of the blood occurs without the need to open the main unit or move the blood sample from one place to another.

The filtration by gravity does not require any clotting or centrifugation of the blood, thereby avoiding the need for any source of energy. Moreover, it allows preserving the clotting factors of the blood, including the platelets, thereby allowing production of plasma rathen than serum, if required.

The filtered serum (or plasma) then flows in a distribution chamber which can then distribute serum (or plasma) in equal quantities to a plurality of vials.

The connectors between the vials and the main unit ensure that the serum is not exposed to the atmosphere, since the connectors are normally closed, and only open when the vials are in fluid-tight contact with the main unit.

Therefore once the vials are filled with serum (or plasma) the connectors return to a closed state, thereby preventing any undue contact between the serum and the atmosphere.

### BRIEF DESCRIPTION OF THE DRAWINGS

The features and advantages of the invention will be apparent from the following more detailed description of certain embodiments of the invention and as illustrated in the accompanying drawings, in which:
- Figures 1a and 1b are cut views of a main unit of a system according to different embodiments of the invention.
- Figures 2a and 2b are cut perspective views of a dispensing plate of a main unit according to two different embodiments of the invention,
- Figures 3a and 3b illustrate a first embodiment of connectors between a vial and a main unit, in a first state preventing communication and in a second state allowing fluid-tight communication.
- Figures 4a and 4b illustrate a second embodiment of connectors between a vial and a main unit, in a first state preventing communication and in a second state allowing fluid-tight communication.
- Figures 5a and 5b illustrate a third embodiment of connectors between a vial and a main unit, in a first state preventing communication and in a second state allowing fluid-tight communication.
- Figures 6a and 6b illustrate a fourth embodiment of connectors between a vial and a main unit, in a first state preventing communication and in a second state allowing fluid-tight communication.
- Figures 7a and 7b illustrate an eighth embodiment of connectors between a vial and a main unit, in a first state preventing communication and in a second state allowing fluid-tight communication.
- Figures 8a and 8b illustrate a ninth embodiment of connectors between a vial and a main unit, in a first state preventing communication and in a second state allowing fluid-tight communication.
- Figures 9a to 9g illustrate a tenth embodiment of connectors between a vial and a main unit.
- Figures 10a and 10b illustrate the use of a nozzle designed to open the connector of a vial to dispense a quantity of serum.

### DETAILED DESCRIPTION OF A PREFERRED EMBODIMENT OF THE INVENTION

A system for manufacturing serum or plasma from a blood sample comprises a main unit 1, and a plurality of vials 2 for collecting, storing and delivering to a patient a determined amount of serum (resp. plasma), the vials preferably all having the same capacity.

The serum (resp. plasma) is preferably used as eye-drops for the treatment of the eye pathologies or troubles discussed above.

### Main unit for collection and filtration of blood

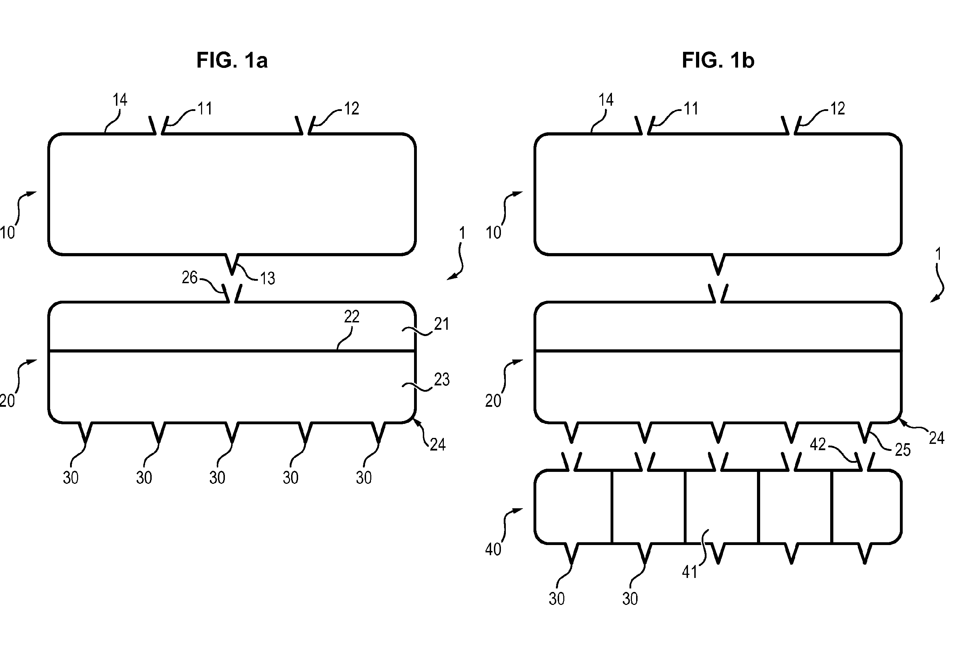

Example embodiments of the main unit 1 are shown on figures 1 a and 1 b.

The main unit 1 comprises a sampling chamber 10 which function is to receive a quantity of blood collected from the patient and hold this quantity before filtration.

In order to receive the blood, the sampling chamber 10 comprises a main access 11. The main access 11 is preferably located on a top wall 14 of the chamber when the latter is in a position of use.

The main access 11 is preferably designed to ensure a fluid-tight connection with a sampling tube, so that during a blood sampling, a sampling tube fitted on the main access 11 can connect directly the sampling chamber to a sampling syringe, thereby ensuring that the sampled blood is not exposed to atmosphere and hence not contaminated. When no tube is connected to the main access, the latter is closed in order to prevent any contamination of the blood inside the chamber.

The main access 11 can for example be a female luer equipped with a luer activated valve or a needle allowing connection to vacuum sampling tubes.

In one embodiment, the sampling chamber 10 may also comprise a secondary access 12, allowing introduction in the sampling chamber of additives for blood supplementation.

The secondary access 12 is disposed on the same wall as the main access 11, which is, when the sampling chamber is in a position of use, the top wall 14 of the latter.

As the main access, the secondary access 12 is also preferably designed to ensure fluid-tight connection to an additive container in order to prevent the blood from being contaminated, and to be closed when no container or tube is connected to the chamber. The secondary access 12 can be for instance a female luer with luer activated valve.

The additives introduced in the sampling chamber 10 through secondary access 12 are preferably chosen according to an eye disease of trouble to cure;

For instance, the additives may preferably comprise sterile saline solution (for instance at a concentration of 0.9% i.e. of 9 g/L of NaCl, and may also comprise at least one of the following components :
- Ofloxacine, ciprofloxacine, tobramycine,
- NGF (Nerve Growth Factor)
- IGF (Insulin-like Growht Factor)
- EGF (Epidermal Growth Factor)
- Compounds inhibiting TGF beta-1 and beta-2 (Transforming Growth Factor)
- Thymosin beta-4
- Compounds inhibiting IL-17 and/or IFN like DHODH (DiHydroOrotata DeHydrogenase) inhibitor
- siRNA targeting Beta 2 Adrenergic Receptor (for the treatment of glaucoma)
- siRNA mediating silencing of capsaicin receptor TRPV1 gene
- Anti-coagulant.

The internal capacity of the sampling chamber 10 is preferably comprised between 20 mL and 100 mL, preferably equal to 30 mL.

The main unit also comprises a filtration chamber 20, in fluid communication with the sampling chamber 10.

Preferably, the filtration chamber 20 and sampling chamber 10 are connectable, though separate units, to allow gentle shaking of the sampling unit in order to mix the blood (as the case may be, with the additives).

Therefore, the sampling chamber 10 also comprises a connector 13 with the filtration chamber 20, to allow transfer of the blood from the sampling chamber to the filtration chamber. The connector 13 is preferably disposed on a wall opposite the wall 14 on which is located the main access. In position of use, the connector 13 is preferably disposed on a bottom wall of the sampling chamber 10.

However, the sampling chamber 10 and the filtration chamber 20 may also not be separated, in which case the connector 13 may be replaced by a stopcock.

The filtration chamber 20 comprises a first compartment 21, a filter 22 and a second compartment 23, the filter 22 separating the first and second compartments.

The first compartment is in fluid communication with the sampling chamber 10 when the latter is connected to the filtration chamber 20. To this end, the filtration chamber 20 may comprise a connector 26, on a wall adjacent to the first compartment, the connector 26 being designed to cooperate with the connector 13 of the sampling chamber, as the case may be, to ensure a fluid-tight connection between the sampling chamber and the first compartment 21.

The connector 26 is for instance on a top wall of the filtration chamber 20 when the latter is in position of use.

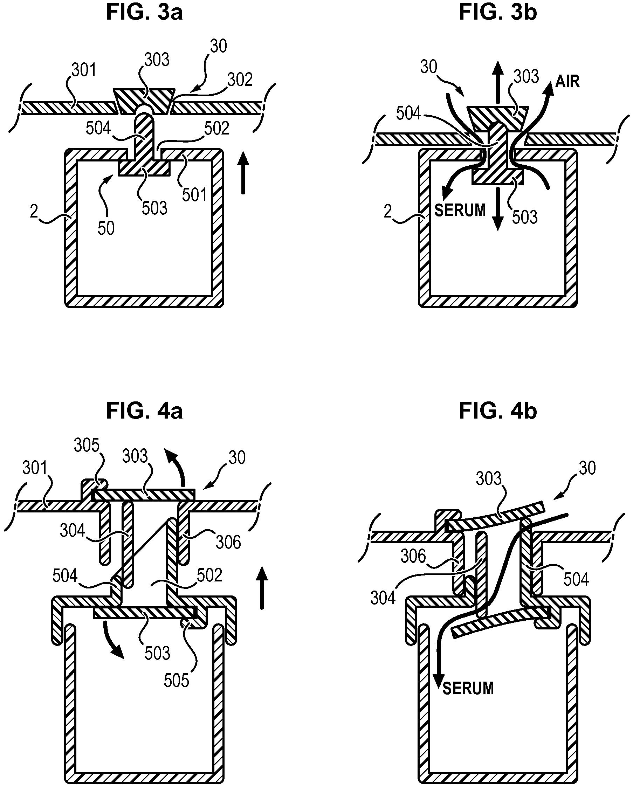

The connectors 13 and 26 of the sampling chamber 10 and the filtration chamber 20 may preferably be designed the same way as connectors 30 and 50 detailed hereinbelow with reference to figures 3a and 3b, 4a and 4b, 5a and 5b, 6a and 6b, 7a and 7b, or 8a and 8b.

The filter 22 is a perforated membrane designed to allow passage of blood serum, its protein content and platelets, but to retain erythrocytes.

As the minimum diameter of erythrocytes is 6,2 µm, and a typical size of platelets is around 4 µm, the cutoff value of the filter (i.e. the dimension of the holes of the filter) is preferably around 5 µm.

The filter 22 is preferably chosen from the range of products sold by Pall Corporation®, for example Asymmetric Polysulfone Membranes of grade GF, GX or GR. Optionally the filter 22 may also be made of glass fiber media.

The surface of the filter is calculated according to the membrane characteristics in µL/cm². Membranes can have filtration capacities comprised between 20µL/cm² and 50 µL/cm². For instance, for a sampling chamber 10 having an internal capacity of about 30 mL, one can select a membrane having a filtration capacity of 50µL/cm² and having a size chosen between 20*20 cm and 25*25 cm,

The second compartment 23 collects the blood after filtration, i.e. the plasma (erythrocytes and other components such as leukocytes have been removed).

The filtration chamber 20 is designed to allow filtration of blood by gravity. In position of use, when the main unit 1 is laying on a generally horizontal surface with the filtration chamber below the sampling chamber, the second compartment extends below the first compartment 21, with the filter 22 extending between the two compartments 21, 23. Blood can therefore flow inside the first compartment by the connector 26, go through the filter and reach the second compartment.

The filtration of blood allows obtaining serum.

Alternatively, the sampled blood can be supplemented with anticoagulant, in which case the filtered blood results in serum in which platelets and other clotting factors are preserved, i.e. plasma.

With the classical serum extraction processes, the same result could not be obtained because blood supplemented with anticoagulant cannot be centrifuged to separate plasma.

The thus obtained plasma is therefore rich in platelets which are known to secrete some of the growth factors (such as platelet-derived angiogenesis factor, platelet-derived epidermal growth factor and platelet factor IV) known to accelerate different aspects of corneal wound healing (see for instance J. L. Alio et al. "Symptomatic Dry Eye Treatment with autologous platelet-rich plasma", Ophthalmic Research 2007; 39:124-129).

Moreover, it is obtained without the need for any external source of energy.

The filtration chamber 20 further comprises a distribution plate 24. The distribution plate 24 is preferably arranged on a bottom wall of the second compartment 23 in position of use, so that, the filtered blood falls from the filter 22 on the distribution plate 24.

The function of the distribution plate 24 is to equally distribute the filtered material into a plurality of volumes to fill corresponding vials 2.

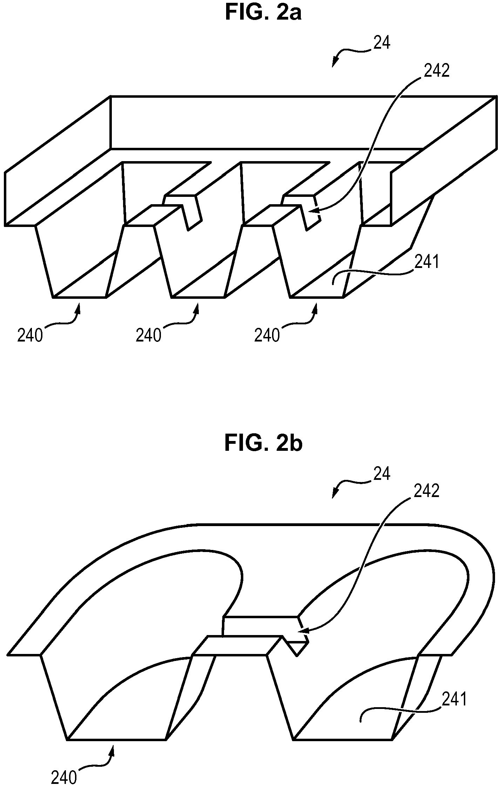

With reference to figure 2a and 2b, the distribution plate 24 comprises a plurality of cavities 240 of equal volume, collecting the filtered blood falling under gravity from the filter 22.

The cavities can have different shapes. Preferably, the transversal section of the cavities (i.e. a section in a plane that is orthogonal to the bottom wall and the filter 22) is a truncated cone with the largest side closest from the filter.

The section of the cavities in a plane parallel to the bottom wall and/or the filter 22 can be either square, rectangular, circular, etc.

Each cavity comprises a bottom wall 241, the bottom walls of all the cavities being preferably coplanar and aligned at a same level to ensure that all the cavities have the same depth. If the cavities also have the same shape and dimensions, they thus have identical volumes.

Preferably, the dispensing plate comprises from 1 to 30 cavities 240, preferably around 10 cavities. Preferably, each cavity has an internal volume comprised between 1 and 10 mL, more preferably comprised between 1,5 and 2,5 mL, more preferably equal to 2 mL.

Moreover, the cavities 240 are preferably interconnected by a series of channels 242, each channel extending from one cavity to an adjacent cavity at a constant height from the bottom walls 241. The height of the channels from the bottom walls 241 defines the maximum volume of a cavity since, when a level of fluid exceeds that of the channel, the fluid overflows into the adjacent cavity by the channel 242.

Therefore the channels 242 ensure that all the cavities have the same capacity and that all the filtrated blood is equally distributed among them.

Each cavity 240 further comprises a connector, arranged at the bottom wall 241.

According to one embodiment shown on figure 1a, each connector 30 is adapted to connect a cavity 240 of the dispensing plate to one of the plurality of vials 2. The structure of the connectors is disclosed in more details below.

According to another embodiment shown on figure 1b, each connector 25 is adapted to connect one cavity 240 of the dispensing plate 24 to another plate 40 of the main unit 1, called supplementation plate.

The supplementation plate 40 can be used to add to the filtrated blood one or more additives, for example from the list detailed above. Indeed, in particular if the sampling chamber 10 has no secondary access 12 for filling in additives, the supplementation can occur after the filtration of blood in the supplementation plate, and not before, in the sampling chamber.

As the filtrated blood is distributed into equal volumes in the dispensing plate 24, the supplementation plate 40 preferably maintains this distribution unchanged. To this end, it comprises a plurality of compartments 41, in equal number as the number of cavities of the dispensing plate 24. Hence it can comprise from 1 to 30 compartments, preferably 10 compartments. Each compartment has a volume preferably greater than that of a cavity 240 of the dispensing plate 24. For instance, each compartment can have a volume comprised between 2 and 10 mL, for instance around 2 or 3 mL.

The supplementation plate 40 further comprises a plurality of connectors 42 to connect each compartment with a cavity of the dispensing plate 24. Preferably the number of connectors 42 equals the number of compartments 41 and the number of cavities of the dispensing plate 24. The connectors 42 are disposed on a top wall of the supplementation plate 24 when the main unit 1 is in position of use.

The connectors 42 are designed to cooperate with the connectors 25 of the dispensing plate 24 to achieve a fluid-tight communication between each one of the cavities 240 and a corresponding compartment 41 of the supplementation plate.

Preferably, the connectors 25 of the dispensing plate and the connectors 42 of the supplementation plate 40 are designed the same way as the connectors 30 and 50 detailed hereinbelow with reference to figures 3a and 3b, 4a and 4b, 5a and 5b, 6a and 6b, 7a and 7b, or 8a and 8b. In use, the supplementation plate 40 is preferably prefilled with the desired amount of additive. Upon connection of the dispensing plate 24 of the filtration unit 20 on the distribution plate 40, the serum or plasma obtained by filtration flows by gravity in each compartment 41 of the supplementation plate, fills said compartment and is mixed with the prefilled additive to result in the appropriate amount of serum (resp. plasma) dose to fill each vial.

In order to fill the vials 2, the supplementation plate further comprises a plurality of connectors 30, each connector being designed to cooperate with a vial's connector 50. The connectors 30 are arranged on a bottom wall of the supplementation plate, when in position of use, that is to say opposite the connectors 42.

The connectors 30 can therefore be positioned at a bottom wall of the supplementation plate 40 or of the dispensing plate 24, according to the chosen embodiment. In the following, the connectors 30 will be designated as the connectors of the main unit.

### Description of the connectors between the main unit and the vials

The structure of the connectors 30 of the main unit and the connectors 50 of the vials 2 will now be described.

The connectors 30, 50 can be implemented in a variety of ways. However, the connectors 30, 50 need to stay in a closed position when the vials and the main unit 1 are separated from one another, and to open only by a specific mechanical action, upon connection, to ensure a fluid-tight connection between the main unit and a vial. By fluid-tight connection, it is understood that the filtrated serum (resp. plasma) can flow from the main unit to the vial, but no other fluid, liquid or gas can penetrate inside the vial.

Preferably, the mechanical action, causing one connector 30 to open, results from the action of bringing closer the opposite connector 50. Preferably, the action of bringing closer the connectors 30, 50 simultaneously opens the two connector 30, 50, and at the same time closes any gap between the main unit 1 and the vial 2, so that the connectors are open only when the main unit 1 and the vial 2 are in contact with each other. This prevents any air from the atmosphere surrounding the vial and the main unit to penetrate inside the vial. Therefore it renders unnecessary the manipulation of the main unit 1 and vial in a clean room laboratory.

Once two connectors are disconnected and the main unit 1 and the vial separated, the connectors 30, 50 return to a closed state.

A plurality of non-limiting embodiments are disclosed in figures 3a to 9e. In the following, a pair of connectors 30, 50 comprising a connector 30 of the main unit and a connector 50 of a vial will be detailed.

### Hole-covering plugs

In some embodiments, the main unit connector 30 comprises a wall 301, which is either the bottom wall of the supplementation plate or that of the dispensing plate. The connector 30 further comprises a through hole 302 arranged inside the wall 301, and a plug 303 closing the hole when the connector 30 is in a closed configuration.

The plug 303 is movable relative to the hole 302 so as to selectively switch the connector 30 from a closed to an open configuration and vice-versa.

Similarly, the vial connector 50 comprises a wall 501, which can be a top wall of the vial 2 when the latter is in position of use, for instance put below the main unit 1, and a through hole 502 arranged in the wall 501.

The vial connector 50 further comprises a plug 503 closing the hole 302 when the connector 50 is in a closed configuration.

The plug 503 is movably mounted relative to the hole so as to allow opening and closing of the connector 50.

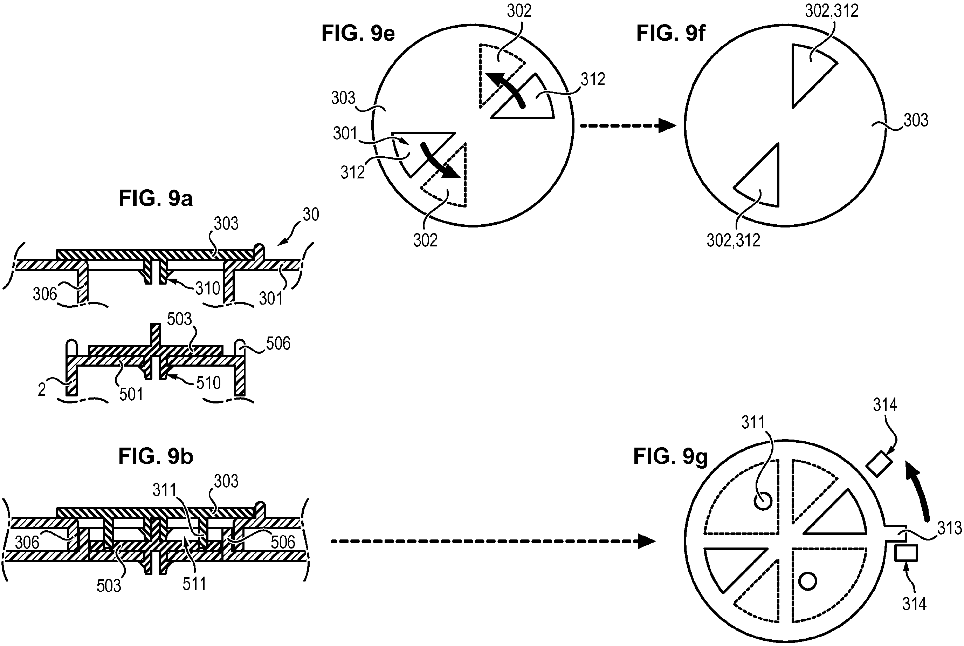

In one embodiment, such as for instance the embodiment of figure 3a, the plug 503 of the vial connector 50 is disposed within the vial. In another embodiment, such as for instance the one shown in figure 9a, the plug may also be outside from the vial.

Similarly, the plug 303 of the main unit connector 30 is preferably within the main unit - that is within the supplementation plate or the dispensing plate - as in figures 3a to 9a. Otherwise, the plug 303 may also be on the other side of the wall 301, outside the main unit.

In one embodiment, shown for instance in figures 3a and 3b, the plug 303 and/or 503 may be mounted in translation relative to the hole 302, 502 of the wall 301, 501. The plug may be urged against the respective hole by a mechanical force, for instance exerted by a spring (not shown), and opens only if a superior force is applied by the opposite connector.

In another embodiment, shown for instance in figures 4a to 7b, the plug 303 and/or 503 may be a flexible plate covering the respective hole 302, 502, and attached to the respective wall 301, 501, by at least one fixation point 305, 505.

In the embodiments shown in figures 4a to 6b, the fixation point 305, 505 may be disposed on a side edge of the plug 303.

The fixation point 305, 505 may be a hook protruding from the wall, in which the plate is inserted (as on the attached drawings). It may alternatively be a pin or any other equivalent means to keep the plug 303 maintained against the wall.

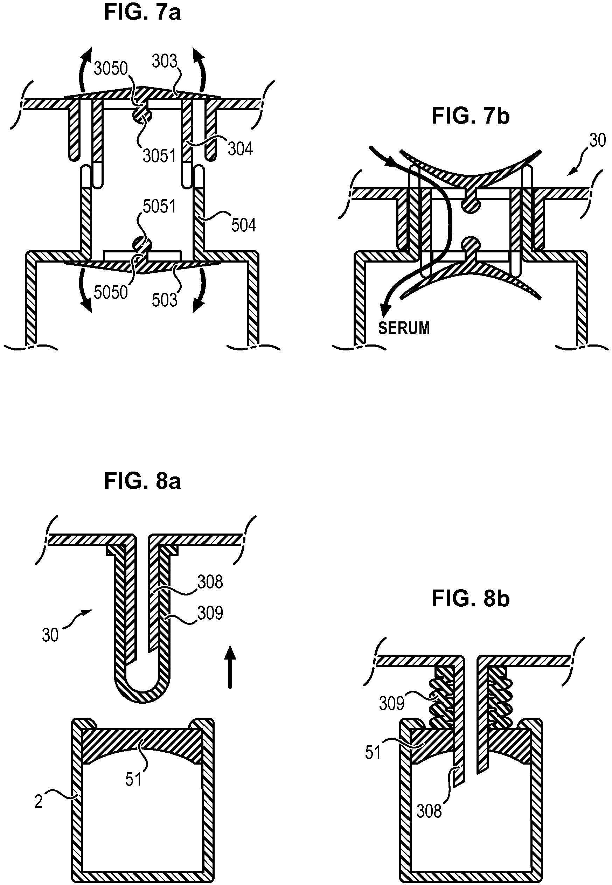

Alternatively, the fixation point 305, 505 may be located in the middle of the plug 303/503, such as on figures 7a and 7b. In this case, the fixation point may be made by the assembly of a secondary through hole 3050, 5050 within the wall, and a protrusion 3051, 5051 linked to the flexible plug, inserted inside the hole to keep the plug linked to the wall.

The connectors 30, 50 are in that case an umbrella valve, and the respective hole 302, 502 (not shown) is circular and surrounds the fixation point of the valve.

### Protruding element

At least one connector of a pair of connectors 30, 50 further comprises a protruding element. In figures 3a and 3b is shown an embodiment in which only one connector has a protruding element 504. The protruding element may either be borne by the connector 30 or the connector 50.

In the case where only one connector of a pair comprises a protruding element 504, said element is preferably affixed to the respective plug 503. Therefore, by bringing the vial closer to the main unit 1, the protruding element 504 comes into contact with the opposite plug 303 closing the opposite hole 302,causing both plugs 303, 503 to move relative to their respective hole 302, 502 and thereby opening the connectors, as shown on figures 3b.

In some embodiments, both connectors 30, 50 of a pair comprise respective protruding elements 304, 504. In that case, the protruding elements 304, 504 can either be borne by the respective plugs 303, 503 or by the respective walls 301, 501.

Moreover, as shown on figures 4a and 4b, one connector of a pair can have a protruding element 304 extending from its plug 303, and the other can have a protruding element 504 extending from its wall 501.

In any case, the protruding elements are designed to open the opposite connector by pushing on the opposite plug only when a fluid-tight contact is established between the vial and the main unit.

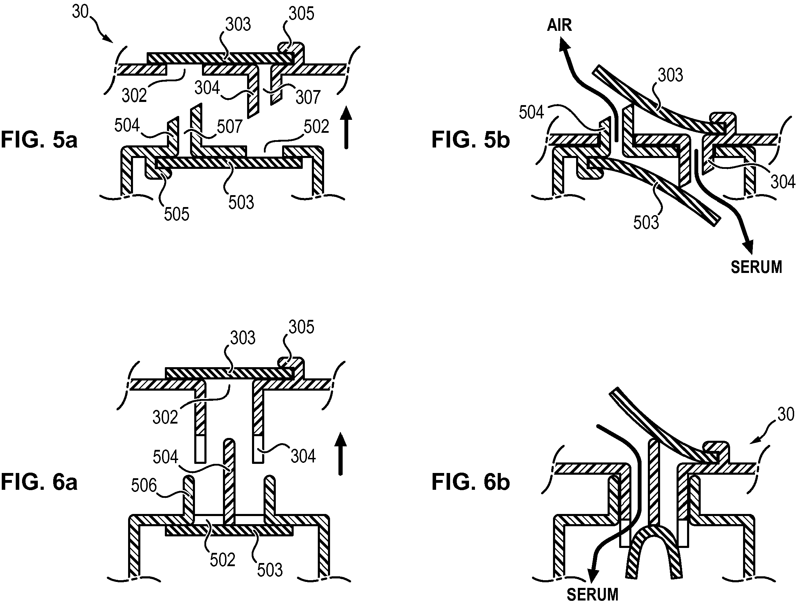

For instance, as shown on figures 4a to 7b, a protruding element 304, 504 can be a hollow tube, and preferably a beveled tube. The base of the tube 304, 504, by which the element is linked to the respective wall 301, 501 surrounds a through hole which may be the hole 302, 502, such as in figures 6a and 6b or a secondary hole 307, 507, such as in figures 5a and 5b.

In this latter case the respective plug 503 preferably covers both holes 502, 507, and the opposite connector is preferably designed to have one hole 302 in which the opposite protruding element 504 can penetrate, and one protruding element 304 able to penetrate in the opposite hole 502. The holes of one connector are thus aligned with the protruding element of the opposite connector and vice-versa.

Preferably, the hole 502, 302 in which the opposite tube 304, 504 penetrates has an internal diameter which is equal to the external diameter of the tube in order to ensure a leak-tight communication between the main unit and the vial.

Therefore, when the free end of the tube 304, 504 reaches the opposite plug 503, 303, it fills the corresponding hole so that fluid can only pass through the tube and not around the tube.

Alternatively, as shown on figures 3a, 3b, 4a, 4b, 6a and 6b the protruding element 304 may be a pin. The pin preferably extends from a corresponding plug of the connector. In that case, in order to ensure a leak-tight communication between the main unit and the vial, the corresponding connector preferably also comprises a protruding panel 306, 506 surrounding the respective pin 304, 504.

The protruding panel 306, 506 is preferably a cylinder having an external diameter equal to the internal diameter of the opposite hole 502, 302.

In a case where both connectors 30, 50 of a pair comprise protruding elements 304, 504, one of which is a pin 504 and the other is a tube 304, as on figures 6a and 6b, preferably one of the tube 304 and the protruding cylinder 506 surrounding the pin 504 is longer than the pin 504. Therefore one of the tube and the cylinder opens the opposite connector 30 before the pin, and closes a channel between the main unit and the vial before the connectors open, thus avoiding any risk of leak.

In a nutshell, the features described above regarding:
- disposition, fixation of a plug and possible movement relative to a respective wall,
- disposition and size of a protruding element
   can all be combined as long as :
   - the connectors only open when a leak-tight connection between the vial and the main unit is achieved, and
   - the connectors return in a closed, leak-tight position, when disconnected.

Preferred embodiment comprising various combinations of the above features are detailed below.

In the embodiment of figures 3a and 3b, the main unit connector 30 comprises a plug 303 mounted in translation relative to the hole 302. The plug has a shape of a truncated cone, and the hole has an internal cross section also in the shape of a truncated cone. In closed configuration, the plug 303 fills the hole 302. It may be urged against the hole by a spring (not shown).

The vial connector 50 comprises a plug 504 covering the hole 502. The plug 504 may also be urged against the hole 502 by a spring (not shown).

The plug 504 of the vial connector 50 comprises a protruding pin 504.

With specific reference to figure 3b, when the vial 2 is brought in contact with the main unit 1, the protruding pin 504 pushes against the plug 303, thereby lifting the plug within the main unit and allowing passage of fluid from the main unit to the vial.

In the embodiment of figures 4a and 4b, the pain unit connector comprises a flexible plug 303 covering the hole 302. The plug is attached to the inner surface of the wall 301 by a hook 305.

The plug 303 bears a protruding pin 304 extending outwardly from the main unit. The wall 301 of the main unit connector also comprises a protruding cylindrical panel 306 surrounding the pin.

The vial connector 50 comprises a flexible plug 504 covering the hole 502. The plug is attached to the inner surface of the wall 501 by a hook 505.

The wall 501 of the vial 2 bears a protruding beveled tube 504 of smaller diameter than the opposite cylindrical panel 306. The internal diameter of the hole 302 of the main unit connector is equal to the external diameter of the beveled tube 504.

The height of the protruding panel 306 is greater that the height difference between the lowest and highest points of the walls forming the beveled tube 504 in order to ensure that, when the highest point of the beveled tube is touching the opposite plug, a channel formed by the beveled tube 504 and the cylindrical panel 306 is closed. A leak-tight connection is thus created between the main unit and the vial.

With specific reference to figure 4b, when the vial 2 is brought in contact with the main unit 1, the protruding pin 304 and the beveled tube 504 urge toward the opposite plugs and cause the plug to twist and open the respective connectors.

In the embodiment of figures 5a and 5b, both connectors 30, 50 comprise a first hole 302, 502, a second hole 307, 507, and a flexible plug 304, 504 covering both holes. The holes of a connector are offset of the same distance as the holes of the other connector.

The plugs 304, 504 are attached on a side edge thereof by respective hooks 305, 505.

The connectors 30, 50 further comprise, extending from the respective wall around the respective second hole 307, 507, a protruding beveled tube 304, 504.

The beveled tube 304, 504 have an external diameter equal to the internal diameter of the opposite first holes 502, 302.

With specific reference to figure 3b, when the vial 2 is brought in contact with the main unit 1, the two protruding tubes 304, 504 penetrate inside the opposite first holes 302, 502, and lift the opposite plug. A first channel formed by:
- the second hole 307 of the main unit connector,
- the protruding tube 304 of the main unit connector, and
- the first hole 502 of the vial connector
allows serum (resp. plasma) to flow from the main unit to the vial.

A second channel formed by:
- the first hole 302 of the main unit connector,
- the protruding tube 504 of the vial connector, and
- the second hole 507 of the vial connector
allows air to flow back from the vial to the main unit.

These two channels can be made at the same time given the equal distance extending between the hole and the protruding tube of each connector.

In the embodiments of figures 6a and 6b, the main unit connector 30 comprises a flexible plug 303 covering the hole 302 and attached to the wall 301 on a side edge by a hook.

The connector 30 also comprises a protruding tube 304.

The vial connector 50 comprises a flexible plug 503 covering the hole 502, and bearing in its middle a protruding pin 504. The pin may be attached to the wall by a flexible arm (not shown) to prevent the pin and plug from falling inside the vial.

The vial connector 50 further comprises a protruding cylindrical panel 506.

With specific reference to figure 6b, when the vial 2 is brought in contact with the main unit 1, the protruding cylinder 304 cooperates with the protruding panel 506 to form a closed channel between the main unit 1 and the vial 2 before or at the time of opening one of the connectors 30. The protruding cylinder 304 then lift the side edges of the opposite plug 503 to open the connector 50, while the pin 504 lifts the opposite plug 303 to open connector 30.

With reference to figures 7a and 7b, the connectors 30, 50 comprise a plug 303, 503 in the shape of an umbrella valve, which is fixed at its center and which peripheral edge can move relative to the respective wall.

The connectors 30, 50 also comprise coaxial protruding cylinders 304, 504. The holes 302, 502 are designed to allow the opposite cylinders to penetrate.

When a vial 2 is brought in contact to the main unit, the walls of the cylinders penetrate through the opposite walls and cause the opposite plugs to distort and open the connectors.

Another embodiment is detailed with reference to figures 8a and 8b. In this embodiment, the main unit connector 20 comprises a needle 308 which is covered by a sheath 309. The sheath may be made of elastomeric material.

The vial connector 50 comprises a septum 51 which can be pierced by the needle. When connecting the vial to the main unit, the needle pierces both the sheath 309 and the septum 51. The needle creates a leak-tight channel from the interior of the main unit 1 to the interior of the vial 2.

In still another embodiment, shown in figures 9a and 9b, each of the main unit connector 30 and the vial connector comprises a circular plug 303, 503, which is rotatably mounted relative to the respective wall 301, 501. Each circular plug 303, 503 is fixed to the respective wall 301, 501 on its center by a fulcrum 310, 510.

Moreover, the circular plugs 303, 503 of the connectors can also be rotationally fixed together so that a rotation of one plug causes the other to rotate. To this end, one of the circular plugs may have pegs 311 (see figure 9b) on a side thereof, extending towards the opposite plug, while the other plug has recesses 511 designed for receiving the pegs. The wall 301 extending between the plugs 303 also has a through groove (not shown) of the shape of an arc in which the pegs 311 can slide.

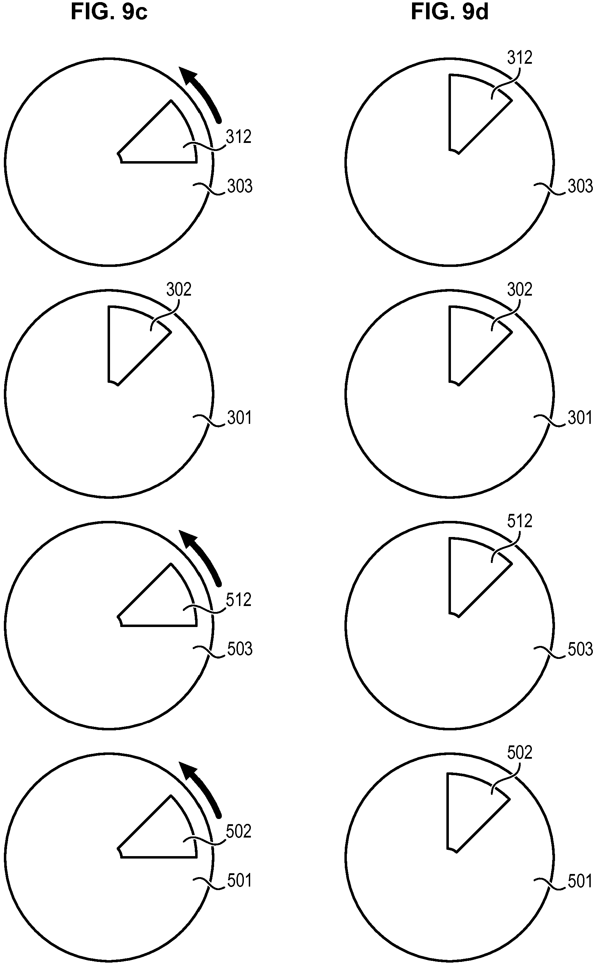

Each plug 303, 503 has a through aperture 312, 512. Each hole can for instance be a sector covering an eighth of a disk. As shown in figure 9c, the holes 312, 512 of the plugs 303, 503 are aligned when the connectors are brought into contact and the pegs received in the recesses.

As also shown in figure 9d, the holes 302, 502 in the walls 301, 501 of the connectors are also aligned when the connectors are brought into contact and the the holes 312, 512 aligned with one another.

Preferably, at least one of the connectors 30, 50 also have a protruding cylindrical panel 306, 506 protruding towards the opposite connector in order to form a leak-tight channel when the connectors are connected.

In order to open the connectors, with reference to figures 9d, 9e and 9f, one moves the vial relative to the main unit 1, in order to bring the holes 302, 502 in alignment with each other and the holes 312, 512 of the plugs in alignment with the holes 302, 502 of the walls.

As shown in figure 9g, the connectors 30, 50 may further comprise a stop, which may be formed by the end of the groove in which the pegs are disposed, or which may be formed, as shown in figure 9g, by a tab 313 of a plug abutting against pawls 314 projecting from the respective wall.

All of the above embodiments disclose a leak-tight connection between the main unit 1 and a plurality of vials 2, thereby suppressing the need of working in a clean room laboratory.

Moreover, in order to use the content of a vial, said vial 2 can be completed by a dispensing nozzle 6 provided separately in a sterile individual packaging, and designed to open the specific connector 50 of the vial which closes the latter.

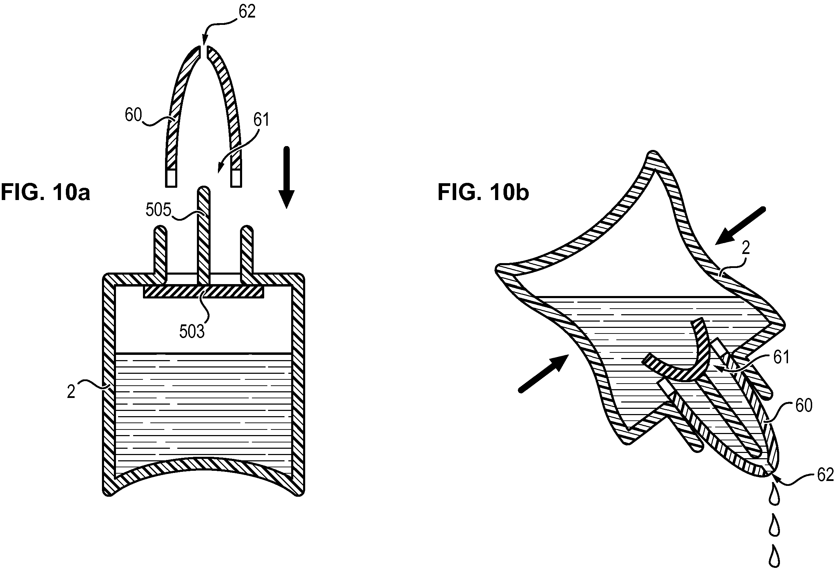

An exemplary embodiment of such a dispensing nozzle is shown in figures 10a and 10b. The dispensing nozzle in particular comprises a mouth 61 which can fit in the hole 502 of the vial and open the connector by distorting the plug 503. An opposite, smaller mouth 62 is open to let serum (resp. plasma) flow in droplets.

## Claims

1. A system for preparing serum or plasma from a blood sample, comprising a main unit (1), comprising
o a sampling chamber (10) for receiving a blood sample, and
o a filtration chamber (20) for filtering the blood sample, the filtration chamber comprising a first compartment (21) in fluid communication with the sampling chamber (10), a second compartment (23), and a filter (22) interposed between the first (21) and second compartments (23),
**characterized in that** it further comprises a plurality of vials (2), each comprising a connector (50), and the main unit (1) comprises a plurality of connectors (30) adapted to cooperate with the vial connectors (50) to achieve a leak-tight communication between the main unit (1) and the vials (2),
and **in that** the filtration chamber (20) is designed to allow blood filtration by gravity when the main unit (1) is in a position of use.

2. A system according to claim 1, wherein a pair of connectors (30, 50), comprising a connector (50) of a vial (2) and a connector (30) of the main unit (1), is designed such that:
- the opening of one connector (30, 50) causes the other connector (50, 30) to open, and
- the opening of a connector (30, 50) occurs only if the vial (2) is in contact with the main unit (1).

3. A system according to claim 1 or 2, wherein each connector (30, 50) of a pair of connectors comprising a vial connector (50) and a main unit connector (30), comprises a wall (301, 501) comprising a through hole (302, 502), and a plug (303, 503) adapted to close said through hole (302, 502), movably mounted relative to the respective wall (301, 501) so as to selectively close or open the respective through hole.

4. A system according to claim 3, wherein at least one plug (303, 503) of the pair of connectors (30, 50) is mounted in translation relative to the respective wall (301, 501).

5. A system according to claim 3 or 4, wherein at least one plug (303, 503) of the pair of connectors (30, 50) is a flexible plate covering the respective hole and fastened to the wall by at least one fixation point (305, 505).

6. A system according to the claim 5, wherein the fixation point (305, 505) is located on a side edge of the flexible plate (303, 503), or on a center point of the flexible plate.

7. A system according to any of claims 3 to 6, wherein at least one connector (30, 50) of the pair of connectors comprises a protruding element (304, 504) extending outwardly from the respective wall (301, 501) and adapted to cause the plug (303, 503) of the other connector to move when the vial (2) is brought in contact with the main unit (1).

8. A system according to claim 7, wherein at least one protruding element (304, 504) of the pair of connectors (30, 50) comprises a pin protruding from a connector's plug (303, 503).

9. A system according to claim 7 or 8, wherein at least one protruding element (304, 504) of the pair of connectors comprises a tube protruding from the respective wall (301, 501).

10. A system according to claim 9, wherein the protruding elements (304, 504) of both connectors (30, 50) of the pair are tubes protruding from the respective walls, and the tubes (304, 504) are offset relative to their respective through hole (302, 502) by an equal distance thereby allowing each tube of a connector to penetrate in the other connector's through hole at the same time.

11. A system according to claim 3, wherein each connector of the pair of connectors (30, 50) comprises a plug (304, 504) having at least one aperture (312, 512), the plug being rotatably mounted with respect to the respective wall (301, 501), allowing selective alignment of the plug aperture with the respective through hole (302, 502), and the plugs having coupling means (311, 511) allowing the rotation of a plug to cause the other plug of the pair to rotate when the vial is brought in contact with the main unit.

12. A system according to claim 1 or 2, wherein each main unit connector (30) comprises a needle (308) and a sheath (309) covering the needle, and each vial connector (50) comprises a septum (51), the needle being adapted to pierce both the sheath and the septum.

13. A system according to claims 1 to 12, wherein the filtration chamber (20) comprises a dispensing plate (24) designed to collect filtrated blood, and the dispensing plate (24) comprises a plurality of cavities (240) of equal capacity, the cavities having coplanar bottom walls (241), and being fluidly connected to each other by communication channels (242) extending at a constant distance from the plan of the bottom walls (241) to ensure equal distribution of a quantity of fluid to the cavities.

14. A system according to claims 1 to 13, wherein the main unit (1) further comprises a supplementation plate (40) comprising a plurality of compartments (41), the supplementation plate bearing the main unit connectors (30) and being connected to the dispensing plate (24) so that each compartment (41) of the supplementation plate is fluidly connected with a cavity (240) of the dispensing plate (24).

15. A system according to any of the preceding claims, wherein, when the main unit is in position of use, the sampling chamber (10) extends above the filtration chamber (20), the first compartment (21) extends above the second compartment (23) and the dispensing plate (24) extends below the filter (22), thereby allowing blood filtration by gravity.

16. A process for manufacturing serum or plasma from a blood sample, the process being carried out in a system according to any of the preceding claims, and comprising:
- a step of providing a blood sample in a sampling chamber (10),
- filtrating the blood sample by gravity in the filtration chamber (20),
- connecting a plurality of vials (2) to the main unit (1), and
- filling the vials with the serum or plasma obtained by blood filtration.
